# NOUVEAU FASCICULE DE BREVET EUROPEEN

(11) **EP 0 862 634 B2**
(45) Date de publication et mention de la décision concernant l'opposition: **01.03.2006**
(45) Mention de la délivrance du brevet: 19.09.2001
(21) Numéro de dépôt: 97935646.6
(22) Date de dépôt: 29.07.1997
(51) Int. Cl.: C12N 15/37, A61K 39/12

(54) **COMPOSITION PHARMACEUTIQUE CONTRE LES TUMEURS ET INFECTIONS A PAPILLOMAVIRUS**
PHARMAZEUTISCHE ZUSAMMENSETZUNG GEGEN DURCH PAPILLOMAVIREN VERURSACHTE TUMOREN UND INFEKTIONEN
PHARMACEUTICAL COMPOSITION FOR TREATING PAPILLOMAVIRUS TUMOURS AND INFECTION

(30) Priorité: 30.07.1996 FR 9609584
(43) Date de publication de la demande: 09.09.1998
(60) Demande divisionnaire: 01109013.1
(73) Titulaire: TRANSGENE S.A., 67000 Strasbourg (FR)
(72) Inventeur: BALLOUL, Jean-Marc, F-67380 Lingolsheim (FR); BIZOUARNE, Nadine, F-67300 Schiltigheim (FR); KIENY, Marie-Paule, F-67100 Strasbourg (FR)
(74) Mandataire: Warcoin, Jacques
(86) Numéro de dépôt international: PCT/FR1997/001412
(87) Numéro de publication internationale: WO 1998/004705

(56) Documents cités:
- EP-A- 0 451 550
- WO-A-90/10459
- WO-A-93/00436
- WO-A-93/02184
- WO-A-94/23037
- WO-A-96/00583
- WO-A-96/11274
- WO-A-96/29091
- WO-A-97/46693
- Vonka et al., Folia Biologica, 42, 73-78 (1996)
- Shah K.V., Howley P.M. "Papillomaviruses" Fields Virology, 2077-2109 (1996)

## Description

La présente invention a pour objet une composition pharmaceutique destinée au traitement ou la prévention des lésions associées aux papillomavirus et plus particulièrement aux papillomavirus humains (HPV) de type 16, 18, 31, 33 et 45

Les papillomavirus sont des virus à ADN possédant un génome circulaire d'environ 7900 paires de bases entouré par une capside protéique. Un certain nombre de types de papillomavirus bovins (BPV), humains (HPV) ont été identifiés et leur génome séquencé (Pfister, 1987, in *The papovaviridae : The Papillomaviruses* (édition Salzman et Howley) Plenum Press, New York, p 1-38). Il comprend une région précoce et une région tardive. La région tardive contient deux cadres de lecture L1 et L2 qui codent pour les composants majeurs de la capside. La région précoce contient au moins les cadres de lecture E1, E2, E4, E5, E6 et E7. Les produits d'expression E1 et E2 régulent la réplication virale et l'expression des gênes viraux alors que ceux des régions E5, E6 et E7 sont impliqués dans les processus de transformation oncogénique des cellules infectées En effet, il a été montré expérimentalement que la protéine E5 de BPV-1 peut *in vitro* transformer des cellules (Schlegel et al., 1986, Science *233*, 464-467). Les protéines E6 de BPV-1 et E7 de HPV-16 sont impliquées dans l'induction et le maintien de la transformation oncogène. Le pouvoir transformant de E7 a été démontré pour HPV-16 et HPV-18 (Kanda et al., 1988, J. Virol. 62, 610-613, Vousden et al., 1988, Oncogene Res. *3*, 1-9 ; Bedell et al., 1987, J. Virol. *61*, 3635-3640). II n'a pas été mis en évidence de fonction à E4.

Chez l'homme, les HPV sont associés à des pathologies allant de l'infection bénigne de la peau aux verrues et aux tumeurs malignes. Ces virus sont hautement spécifiques des épithélium de l'épiderme des voies génitales, orales et respiratoires. Les données épidémiologiques suggèrentfortement le rôle de certaines souches dans le cancer du col de l'utérus et des voies basses, en particulier les HPV-16 et 18 et à un moindre degré les HPV-31, 33 et 45. Le cancer du col utérin est la deuxième cause de cancer féminin dans le monde. Selon l'OMS, 460000 nouveaux cas sont répertoriés chaque année dont 200000 cas au moins sont clairement associés aux HPV. Toute une série d'études démontre le rôle transformant de ces virus, leur intégration spécifique dans le génome des cellules néoplasiques, leur activité génique dans les cellules cancéreuses et l'importance de l'expression des gènes précoces E6 et E7 dans le maintien du phénotype malin des cellules néoplasiques HPV positives (Monsenego, J. Impact Medecin, 11 mars 1994).

Les pathologies associées aux virus HPV posent un problème thérapeutique du fait de leur nature persistante et récurrente. De nombreuses approches ont déjà été utilisées dans le traitement de ces maladies comme la chirurgie, la chimiothérapie, les agents antiviraux et l'immunothérapie.

A cet égard, le brevet européen EP 0 462187 décrit une approche de vaccination utilisant les gênes précoces des papillomavirus pour établir une immunité à l'égard des tumeurs résultant de l'intégration du génome HPV dans l'ADN cellulaire et dans lesquelles les protéines de la capside ne sont plus exprimées. La demande WO 93/02184 enseigne une approche thérapeutique basée sur l'utilisation des antigènes de capside à titre d'agents immunogénes. Ces documents ne suggèrent pas la possibilité d'associer l'effet préventif procuré par les polypeptides précoces et l'effet curatif conféré par les polypeptides tardifs des papillomavirus pour générer des compositions adaptées à l'ensemble des pathologies graves dues aux HPV. Par ailleurs, au cours des dernières années, il a été proposé d'utiliser des polypeptides ayant une activité immunostimulatrice dans le but d'activer les cellules T avec un résultat plus ou moins bénéfique selon les pathologies ciblées (voir par exemple WO 96/11279).

Par ailleurs, WO93/00436 et WO94/23037 décrivent les propriétés immunogènes de la protéine L2 de BPV qui peuvent être mises à profit pour le traitement ou la prévention des tumeurs à papillomavirus. Une formulation particulière associe les protéines L2 et E7 de BPV-4 produites par voie recombinante dans *E. coli.* II est à noter que l'activité anti-tumorale procurée par l'association L2 + E7 est similaire à celle obtenue avec L2 seule.

WO96/11274 décrit des compositions pharmaceutiques pour la prévention ou le traitement des infections à papillomavirus obtenues par l'expression du polypeptide L1 et d'un polypeptide de fusion L2-E7 dans le système baculovirus. Les VLPs (pour *Virus Like Particles*) chimères présentant le polypeptide E7 à leur surface sont produites dans les cellules d'insectes Sf 9 et purifiées par centrifugation sur gradient de sucrose. Les lapins immunisés par ces particules chimères génèrent des anticorps contre les polypeptides viraux L1 et E7. WO96/11274 divulgue également des VLPs présentant une molécule immunostimulatrice à leur surface et illustre son insertion dans la région de L2 exposée à l'extérieur de la capside.

Enfin, WO96/260912 et WO97/46693 décrivent des compositions pharmaceutiques destinées au traitement des infections ou tumeurs à papillomavirus comprenant un polypeptide originaire de la région précoce et/ou un polypeptide originaire de la région tardive du génome d'un papillomavirus en association avec une protéine ayant une activité immunostimulatrice. En particulier, WO96/29091 décrit l'effet bénéfique de l'interleukine-12 pour le traitement des lésions associées au papillomavirus et envisage la combinaison de l'interleukine-12 avec un antigène de papillomavirus sous la forme soit de compositions polypeptides, soit de vecteurs recombinant exprimant lesdits polypeptides, et WO97/46693 décrit des capsides vides VLPs formées par l'auto-assemblage des polypeptides tardifs L1 ou L1+L2 à titre de véhicule de transfert d'un matériel génétique codant pour une protéine d'intérêt. Les VLPs sont mis à profit pour délivrer *in vivo* un matériel génétique d'intérêt, par exemple codant pour une cytokine telle que l'interleukine-2, un polypeptide ayant une activité co-stimulatrice, telle que B7.1 et B7.2 ou encore les polypeptides précoces d'un papillomavirus.

La présente invention concerne plus précisément une préparation basée sur un mélange d'antigènes originaires des régions précoces et tardives d'un papillomavirus ou un vecteur les exprimant simultanément, dans le but d'établir une immunité durable à l'encontre des cellules infectées. Les candidats vaccins proposés dans le cadre de la présente invention peuvent être utilisés à titre préventif (immunoprophylaxie) pour limiter le développement ou la propagation de l'infection virale aux tissus voisins ou à titre curatif (immunothérapie) pour empêcher ou réduire une évolution tumorale chez les patientes infectées. L'utilisation des antigènes de capside va induire la production d'anticorps contre les épitopes antigèniques localisés à la surface des particules virales empêchant l'infection de s'établir durablement. L'usage des protéines précoces va permettre d'induire une immunité à l'encontre des cellules infectées après intégration de l'ADN viral.

La présente invention fournit également une préparation associant un polypeptide originaire d'un papillomavirus et une molécule immunostimulatrice. Un des avantages d'une telle composition est qu'elle combine l'immunité spécifique induite par les antigènes viraux et l'immunité aspécifique induite par la molécule immunostimulatrice et destinée à renforcer la réponse spécifique.

Le but de la présente invention est de mettre à la disposition du public des compositions pharmaceutiques permettant le traitement des infections à HPV et plus particulièrement les pathologies graves telles que le cancer du col de l'utérus, d'une efficacité améliorée par rapport aux compositions de l'art antérieur.

C'est pourquoi la présente invention a pour objet une composition pharmaceutique destinée au traitement ou à la prévention d'une infection ou tumeur à papillomavirus qui comprend à titre d'agents thérapeutiques un polypeptide originaire de la région précoce E6, un polypeptide originaire de la région précoce E7, un polypeptide originaire de la région tardive L1, un polypeptide originaire de la région tardive L2 d'un papillomavirus et au moins un polypeptide ayant une activité immunostimulatrice sélectionné parmi le groupe constitué par l'interleukine-2, l'interleukine-7 et les molécules de co-adhésion B7.1 et B7.2;
étant entendu que ledit polypeptide ayant une activité immunostimulatrice est un polypeptide natif et lesdits polypeptides originaires de la région précoce et lesdits polypeptides originaires de la région tardive sont des polypeptides natifs ou des variants caractérisés par au moins une mutation d'un acide aminé. Par "mutation" on entend délétion, insertion et/ou substitution d'un acide aminé. De manière plus particulière, un polypeptide en usage dans le cadre de la présente invention a notamment une séquence en acides aminés dont le degré de similarité avec la séquence de la protéine native est supérieur à 75 %, avantageusement, supérieur à 85 % et, de préférence, supérieur à 95 %. Le degré de similarité peut être aisément calculé à l'aide d'un programme ordinateur approprié ou en alignant les séquences de manière à obtenir le degré maximal d'homologie et en comptabilisant le nombre de positions dans lesquelles les acides aminés des deux séquences se retrouvent à l'identique par rapport au nombre total de positions La séquence des génomes de HPV-16 et de HPV-18 est divulguée dans Genebank aux numéros d'accession K02718 et X05015 respectivement.

Comme rappelé précédemment, le génome des virus de la famille des papillomavirus, en particulier BPV et HPV, code pour au moins 8 polypeptides, deux polypeptides tardifs L1 et L2 composant la capside virale et 6 polypeptides précoces (E1, E2, E4, E5, E6 et E7) impliqués dans la régulation, le maintien du génome viral et la transformation des cellules infectées.

Dans le cas des protéines E6 et E7, il peut être avantageux d'avoir recours à un variant non oncogène muté au niveau des régions impliquées dans le processus de transformation des cellules infectées. De tels variants sont décrits dans la littérature (Munger et al., 1989, EMBO J. 8, 4099-4105; Crook et al., 1991, Cell *67*, 547-556 ; Heck et al., 1992, Proc. Natl. Acad. Sci. USA 89, 4442-4446 ; Phelps et al., 1992, J. Virol. 66, 2418-2427).

Par "immunostimulatrice", on entend la capacité à stimuler une réponse immunitaire humorale en activant les lymphocytes B afin d'amplifier la production d'anticorps dirigés contre les antigènes de papillomavirus ou à stimuler une réponse immunitaire à médiation cellulaire en activant les lymphocytes T afin de déclencher une réponse cytotoxique significative à l'encontre de cellules tumorales ou infectées par un papillomavirus. A titre indicatif, l'immunostimulation peut être évaluée en modèle animal (par comparaison du taux de rejet dans un animal auquel on a greffé une tumeur exprimant l'antigène cible, ceci en présence et en absence de l'immunostimulateur). D'une manière plus générale, les moyens pour mettre en évidence une immunostimulation sont indiqués dans Roitt (in *Immunology*, 4th edition, Moby Ltd).

On peut utiliser une molécule immunostimulatrice native telle que trouvée dans un mammifère et, en particulier chez l'homme.

Une composition préférée selon l'invention comprend :
(1) un polypeptide originaire de la région E6, un polypeptide originaire de la région E7, un polypeptide originaire de la région L1, un polypeptide originaire de la région L2 d'un papillomavirus et un polypeptide dérivé de l'interleukine-2,
(2) un polypeptide originaire de la région E6, un polypeptide originaire de la région E7, un polypeptide originaire de la région L1, un polypeptide originaire de la région L2 d'un papillomavirus et un polypeptide dérivé de la molécule B7.1, ou
(3) un polypeptide originaire de la région E6, un polypeptide originaire de la région E7, un polypeptide originaire de la région L1, un polypeptide originaire de la région L2 d'un papillomavirus, un polypeptide dérivé de la molécule B7.1 et un polypeptide dérivé de l'interleukine-2.

Etant donné les observations rappelées plus haut sur la fréquence d'infection par certains types d'HPV dans les cas de cancer du col de l'utérus, une composition selon l'invention comprend un polypeptide originaire d'un papillomavirus à risque, de type HPV-16, HPV-1 8, HPV-31, HPV-33 et/ou HPV-45 et en particulier du virus HPV-16. Bien entendu, dans le cas où la composition inclut plusieurs antigènes de papillomavirus, ceux-ci peuvent être d'une origine commune ou différente.

D'une manière générale, un polypeptide originaire d'un papillomavirus ou ayant une activité immunostimulatrice peut être produit par les méthodes conventionnelles de synthèse chimique ou bien par les techniques de l'ADN recombinant (voir par exemple Maniatis et al., 1989, *Laboratory Manual,* Cold Spring Harbor, Laboratory Press, Cold Spring Harbor, NY). Plus particulièrement, un procédé de préparation comprend l'acte de cultiver une cellule transformée par un fragment d'ADN codant pour le polypeptide en question pour générer une cellule productrice et l'acte de récolter ledit polypeptide à partir de la culture. La cellule productrice peut être d'une origine quelconque et sans limitation, une bactérie, une levure ou bien une cellule de mammifère, dans la mesure où le fragment d'ADN considéré est soit intégré dans son génome soit intégré dans un vecteur d'expression approprié capable de se répliquer. Bien entendu, le fragment d'ADN est placé sous le contrôle de signaux de transcription et de traduction permettant son expression dans la cellule productrice. Vecteurs d'expression et signaux de contrôle sont connus de l'homme du métier.

La présente invention vise également une composition pharmaceutique destinée au traitement ou à la prévention d'une infection ou tumeur à papillomavirus qui comprend à titre d'agent(s) thérapeutique(s) un ou plusieurs vecteur(s) recombinant(s) dérivant d'un poxivrus dans le(s)quel(s) sont insérés des fragments d'ADN codant pour :
(1) un polypeptide originaire de la région précoce E6, un polypeptide originaire de la région précoce E7, un polypeptide originaire de la région L1, un polypeptide originaire de la région L2 d'un papillomavirus, ou
(2) au moins un polypeptide originaire de la région précoce d'un papillomavirus, au moins un polypeptide originaire de la région tardive d'un papillomavirus et au moins un polypeptide ayant une activité immunostimulatrice sélectionné parmi le groupe constitué par l'interleukine-2, l'interleukine-7 et les molécules de co-adhésion B7.1 et B7.2,
lesdits fragments d'ADN étant placés sous le contrôle des éléments nécessaires à leur expression dans une cellule ou un organisme hôte.

Selon cette alternative par ailleurs préférée, l'agent thérapeutique est un vecteur dans lequel sont insérés les fragments d'ADN codant pour les polypeptides originaires d'un papillomavirus ou immunostimulateurs tels que définis précédemment. Ce type de composition présente l'avantage d'une production bon marché et d'une grande stabilité dans des conditions d'environnement variées. En particulier, les conditions de conservation sont moins contraignantes.

Les fragments d'ADN codant pour un polypeptide originaire d'un papillomavirus peuvent être obtenus par clonage, par PCR (Polymerase Chain Réaction) ou par synthèse chimique selon les techniques conventionnelles communément en usage à partir de cellules papillomavirus positives obtenues de patients ou de collections.

Le gène codant pour un polypeptide ayant une activité immunostimulatrice peut également être isolé selon les techniques standards à partir du génome d'une cellule (type génomique) ou des ARN messagers d'une cellule dans laquelle il est exprimé (type ADN complémentaire). Par ailleurs, le gène en question peut coder pour (i) une molécule soluble, soit intracellulaire soit secrétée dans le milieu extérieur ou (ii) une molécule ancrée dans la membrane et donc présente à la surface des cellules qui l'expriment.

Le vecteur viral recombinant selon l'invention dérive d'un poxvirus et, notamment, d'un poxvirus aviaire, tel que le poxvirus du canari, d'un fowlpox ou d'un virus de la vaccine, ce dernier étant préféré. Parmi tous les virus de la vaccine envisageables dans le cadre de la présente invention, on choisit de préférence les souches Copenhague, Wyeth et Ankara modifiée (MVA pour Modified Vaccinia Virus Ankara). Les conditions générales d'obtention d'un virus de la vaccine capable d'exprimer un gène hétérologue sont enseignées dans le brevet européen EP 83 286 et la demande EP 206 920. Quant au virus MVA, il est plus particulièrement décrit dans (Mayr et al., 1975, Infection 3, 6-14 ; Sutter et Moss, 1992, Proc. Natl. Acad. Sci. USA 89, 10847-10851).

Bien entendu, dans le cadre de la présente invention, les fragments d'ADN sont placés sous le contrôle des éléments nécessaires à leur expression. Ceux-ci incluent les éléments appropriés de régulation de la transcription ainsi que des signaux d'initiation et de terminaison de la traduction. Le promoteur revêt une importance particulière. D'une façon générale, on aura recours à un promoteur fonctionnel dans l'organisme ou la cellule hôte que l'on veut traiter et adapté au vecteur employé. En outre, il peut être modifié de manière à contenir des séquences régulatrices, par exemple un élément activateur de la transcription ou des séquences répondant à certains signaux cellulaires. A cet égard, il peut être avantageux d'utiliser un promoteur tissu-spécifique puisque les lésions associées aux papillomavirus sont localisées au niveau des voies génitales ou un promoteur répondant à des signaux spécifiquement tumoraux (par exemple activé en présence de facteurs de croissance généralement surexprimés par les cellules tumorales) afin de limiter l'expression aux seules cellules tumorales.

Parmi les promoteurs envisageables dans le cadre de l'invention, on peut citer les promoteurs SV40 (Virus Simian 40), HMG (Hydroxy-Methyl-Glutaryl-coenzyme A), TK (Thymidine Kinase), CMV (cylomégalovirus), RSV (Rous Sarcoma Virus), le MLP (Major Late Promoter) de l'adénovirus adaptés au vecteur adénoviraux et le LTR du Mo-MLV (Moloney Murine Leukemia Virus) plus spécifiques aux vecteurs rétroviraux. Lorsque le vecteur dérive d'un poxvirus, de préférence le promoteur sera le promoteur d'un gène du poxvirus utilisé, par exemple le promoteur du gène codant pour la protéine 7,5K, H5R, TK ou K1L du virus de la vaccine. Ces derniers sont décrits dans la littérature et peuvent être clonés à partir du génome viral par les techniques classiques.

Par ailleurs, les éléments nécessaires à l'expression peuvent également comporter des séquences améliorant l'expression ou le maintien dans la cellule hôte (intron, séquence signal, séquence terminatrice de la transcription, site d'initiation de la traduction, séquences modifiant la présentation du polypeptide aux cellules du système immunitaires de l'hôte....). Cependant, s'agissant d'un vecteur dérivé d'un poxvirus, on évitera l'emploi d'introns.

Une composition selon l'invention peut être obtenue soit avec plusieurs vecteurs recombinants exprimant chacun un polypeptide donné soit avec un seul vecteur exprimant les fragments d'ADN correspondant aux polypeptides choisis placés sous le contrôle d'éléments indépendants ou communs. Selon cette dernière option, on peut avoir recours à des séquences permettant d'initier la traduction de manière interne (IRES) ou à des fusions en phase des différents gènes.

Les conditions générales d'obtention d'un vecteur recombinant en usage dans la présente invention sont largement décrits dans l'état de la technique. S'agissant d'un vecteur poxviral, on peut se référer au brevet européen EP 83 286. Ces conditions sont applicables aux autres virus acceptables comme vecteur qui possèdent une région génomique non essentielle dans laquelle les blocs d'expression peuvent être incorporés. Bien entendu, ils peuvent être insérés dans le même locus ou un locus différent. Par exemple, lorsque l'on utilise un virus de la vaccine de la souche Copenhague, le site d'insertion préféré est le locus TK et/ou le locus K1L. L'insertion au niveau du gène viral TK a pour effet d'inactiver celui-ci et ainsi faciliter la sélection des recombinants. Dans le cadre d'un virus de la vaccine de la souche MVA, l'insertion des gènes de papillomavirus et immunostimulateurs peut être réalisée au sein d'une des excisions I à VI et, de préférence, la zone d'excision II ou III (Meyer et al., 1991, J. Gen. Virol. 72, 1031-1038 ; Sutter et al., 1994, Vaccine 12, 1032-1040).

Conformément aux buts poursuivis par la présente invention, un vecteur recombinant peut en outre comprendre un bloc d'expression d'un gène marqueur de sélection afin de faciliter les étapes d'isolement et de purification du virus recombinant. On peut citer notamment le gène Neo conférant la résistance à l'antibiotique G418, le gène *pac* de résistance à la puromycine, le gène TK du virus de l'herpès simplex de type 1 (HSV-1) qui confère la sensibilité à certains analogues de nucléosides tels que le ganciclovir ou l'acyclovir, les gènes bactériens *Lac*Z codant pour la β-galactosidase et *gus A* codant pour la β-glucuronidase. Ces deux derniers marqueurs enzymatiques permettent de repérer les virus recombinants par coloration en présence des substrats X-Gal (5-bromo-4-chloro-3-indolyl-β-D-galactopyranoside) et XglcA (5-bromo-6-chloro-3-indolyl-β-D-glucoronide) respectivement.

Une composition pharmaceutique selon l'invention en vue du traitement ou la prévention d'une infection ou tumeur à papillomavirus, comprend un ou plusieurs vecteur(s) recombinant(s) dérivé(s) d'un virus de la vaccine dans le(s)quel(s) sont insérés :
(1) au moins un fragment d'ADN codant pour au moins un polypeptide de la région tardive d'un papillomavirus et un fragment d'ADN codant pour la protéine E6 d'un papillomavirus, un fragment d'ADN codant pour là protéine E7 d'un papillomavirus et un fragment d'ADN codant pour la molécule B7.1,
(2) au moins un fragment d'ADN codant pour au moins un polypeptide de la région tardive d'un papillomavirus et un fragment d'ADN codant pour la protéine E6 d'un papillomavirus, un fragment d'ADN codant pour la protéine E7 d'un papillomavirus et un fragment d'ADN codant pour l'interleukine-2,
(3) au moins un fragment d'ADN codant pour au moins un polypeptide de la région tardive d'un papillomavirus et un fragment d'ADN codant pour la protéine E6 d'un papillomavirus, un fragment d'ADN codant pour la protéine E7 d'un papillomavirus, un fragment d'ADN codant pour la molécule B7.1 et un fragment d'ADN codant pour l'interleukine-2,
(4) un fragment d'ADN codant pour la protéine E6 d'un papillomavirus, un fragment d'ADN codant pour la protéine E7 d'un papillomavirus, un fragment d'ADN codant pour la protéine L1 d'un papillomavirus, un fragment d'ADN codant pour la protéine L2 d'un papillomavirus et un fragment d'ADN codant pour la molécule B7.1,
(5) un fragment d'ADN codant pour la protéine E6 d'un papillomavirus, un fragment d'ADN codant pour la protéine E7 d'un papillomavirus, un fragment d'ADN codant pour la protéine L1 d'un papillomavirus, un fragment d'ADN codant pour la protéine L2 d'un papillomavirus et un fragment d'ADN codant pour l'interleukine-2, ou
(6) un fragment d'ADN codant pour la protéine E6 d'un papillomavirus, un fragment d'ADN codant pour la protéine E7 d'un papillomavirus, un fragment d'ADN codant pour la protéine L1 d'un papillomavirus, un fragment d'ADN codant pour la protéine L2 d'un papillomavirus, un fragment d'ADN codant pour la molécule B7.1 et un fragment d'ADN codant pour l'interleukine-2.

En revanche, une composition pharmaceutique selon l'invention plus particulièrement destinée à des fins d'immunoprophylaxie, comprend un ou plusieurs vecteur(s) recombinant(s) dérivé(s) d'un virus de la vaccine dans le (s)quel(s) sont insérés: au moins un fragment d'ADN codant pour au moins un polypeptide originaire de la région précoce d'un papillomavirus et :
(1) un fragment d'ADN codant pour la protéine L1 d'un papillomavirus, un fragment d'ADN codant pour la protéine L2 d'un papillomavirus et un fragment d'ADN codant pour la molécule B7.1,
(2) un fragment d'ADN codant pour la protéine L1 d'un papillomavirus, un fragment d'ADN codant pour la protéine L2 d'un papillomavirus et un fragment d'ADN codant pour l'interleukine-2, ou
(3) un fragment d'ADN codant pour la protéine L1 d'un papillomavirus, un fragment d'ADN codant pour la protéine L2 d'un papillomavirus, un fragment d'ADN codant pour l'interleukine-2 et un fragment d'ADN codant pour la molécule B7.1.

Une composition selon l'invention peut être préparée selon les procédés connus dans le domaine des vaccins et les doses applicables peuvent varier dans une large gamme. Elles sont fonctions notamment des polypeptides et du virus employés, de la pathologie à traiter, de l'état du patient et d'autres paramètres qui peuvent être évalués par le clinicien Cependant, en général, la dose de virus par kilo sera de 10⁴ à 10¹¹, avantageusement de 10⁶ à 10¹⁰ et, de préférence de 10⁷ à 10⁹ unités formant des plages (ufp) lorsque l'agent thérapeutique est un vecteur viral et de 0,05 à 500 mg, avantageusement de 0,5 à 200 mg et, de préférence de 1 à 100 mg lorsque l'agent thérapeutique est d'origine polypeptidique.

Une composition selon l'invention peut être administrée selon n'importe quelle voie conventionnelle d'administration, en particulier par voie intraveineuse, intramusculaire, sous-cutanée ou sous-épithéliale ou bien par scarification. Dans le cas d'une tumeur accessible, il est également possible de recourir à une injection directe dans le site ou à proximité de la tumeur ou à une application topique. A titre de vaccin, une composition selon l'invention peut être administrée selon les pratiques courantes dans le domaine, par exemple en dose unique ou répétée une ou plusieurs fois après un certain délai d'intervalle. Par contre dans le cadre d'un traitement curatif, elle peut être administrée fréquemment pendant une période suffisante pour que le traitement soit efficace. Lorsque l'agent thérapeutique est un vecteur viral, ce virus est de préférence sous forme vivante S'agissant d'un vecteur poxviral, on préférera employer une souche atténuée comme la souche MVA ou la souche Copenhague thymidine kinase négative. Enfin un vecteur viral recombinant peut être atténué par un traitement chimique approprié connu de l'homme du métier. Toutefois, on peut aussi envisager d'injecter un vecteur recombinant tué.

Selon un mode de mise en oeuvre préféré, une composition pharmaceutique selon l'invention comprend une quantité thérapeutiquement efficace d'un vecteur recombinant en association avec un support acceptable d'un point de vue pharmaceutique Le support est choisi de manière à permettre son administration par injection à l'homme ou à l'animal. Elle peut également comprendre un véhicule, un diluant et/ou un adjuvant et se présenter sous forme liquide ou lyophilisée.

La présente invention concerne également une composition pharmaceutique selon l'invention, à titre de médicament pour le traitement ou la prévention du cancer du col de l'utérus, d'une dysplasie du col de bas grade et d'une infection à papillomavirus.

Enfin, la présente invention a également trait à une méthode de traitement ou de prévention des pathologies citées ci-dessus, selon laquelle on administre à un individu ayant besoin d'un tel traitement une quantité efficace d'un point de vue pharmaceutique d'un mélange de polypeptide ou d'un vecteur recombinant en usage dans la présente invention.

La présente invention est illustrée par référence aux Figures suivantes

La Figure 1 est une représentation schématique du vecteur pTG5021 permettant le transfert au locus TK de la vaccine Copenhague des gènes tardifs L 1 et L2 de HPV-16 placés sous le contrôle du promoteur p7,5K, les deux cassettes étant en orientation opposée l'une par rapport à l'autre.

La Figure 2 est une représentation schématique du vecteur pTG5065 permettant le transfert au locus K1L de la vaccine Copenhague des gènes précoces E6 et E7 de HPV-16 placés sous le contrôle du promoteur pH5R (les deux cassettes étant en orientation opposée l'une par rapport à l'autre), du gène IL-2 humain (IL2h) placé sous le contrôle du promoteur p7,5K et du gène marqueur LacZ (btaGAL) placé sous le contrôle du promoteur pK1L.

La Figure 3 illustre de manière schématique la stratégie qui peut être employée pour introduire les gènes tardifs L1 et L2 de HPV-16 au sein de la zone d'exclusion II d'un virus de la vaccine MVA, les bras de recombinaison gauche et droit étant indiqués (BRG2 et BRD2) respectivement.

### EXEMPLES

La présente invention est plus complètement décrite, sans pour autant être limitée, à l'aide des exemples suivants.

Les constructions décrites ci-dessous sont réalisées selon les techniques générales de génie génétique et de clonage moléculaire, détaillées dans Maniatis et al., (1989, *supra)* ou selon les recommandations du fabricant lorsqu'on utilise un kit commercial. La mutagénèse dirigée *in vitro* par oligonucléotides synthétiques est effectuée à l'aide du kit distribué par Amersham. Les techniques d'amplification par PCR sont connues de l'homme de l'art (voir par exemple PCR Protocols-A guide to methods and applications, 1990, edité par Innis, Gelfand, Sninsky et White, Academic Press Inc) S'agissant de la réparation des sites de restriction, la technique employée consiste en un remplissage des extrémités 5' protubérantes à l'aide du grand fragment de l'ADN polymérase I d'*E. coli* (Klenow).

Les étapes de clonage, les bactériophages M 13 recombinants sont multipliés sur la souche *E. coli* NM522 (Stratagène) dans un milieu minimum gélosé (agar 7,5 %) ou dans un milieu riche LBM liquide. Les plasmides recombinants portant le gène de résistance à l'ampicilline sont repliqués dans les souches *E. coli* C600 (Stratagéne), BJ 5183 (Hanahan, 1983, J Mol. Biol. *166, 557-580)* et NM522 sur milieu gélosé ou liquide supplémenté de 100 µg/ml d'antibiotique. On utilise préférentiellement la souche BJ5183 lorsque le clonage est effectué par recombinaison homologue (Bubeck et al., 1993, Nucleic Acid Res. 21, 3601-3602).

La construction des virus de la vaccine recombinants est effectuée selon la technologie classique dans le domaine divulguée dans les documents déjà cités et dans Mackett et al, (1982, Proc Natl. Acad. Sci. USA 79, 7415-7419) et Mackett et al (1984, J. Virol. 49, 857-864).

### EXEMPLE 1 Construction du virus de la vaccine Copenhague VVTG5021 &5065 exprimant les gènes E6, E7, L1 et L2 du HPV-16 et le gène humain IL-2

### A. Isolement des gènes L 1 et L2 de HPV16

Les fragments codant pour les protéines L1 et L2 sont isolés par PCR à partir d'ADN génomique de cellules Caski (ATCC 1550) selon les techniques générales de l'art. Le fragment d'amplification portant les séquences L 1 est sous cloné dans le vecteur M13TG130 (Kieny et al., 1983, Gene 26, 91-99), pour donner la construction M13TG8171, La séquence du gène L1 cloné révèle plusieurs mutations par rapport à la séquence contenue dans Genebank (accession K02718) : C à la place d'un A en position 248, C à la place d'un A en position 253, G à la place d'un A en position 537, G à la place d'un C en position 682, G à la place d'un A en position 874, insertion d'un triplet ACT en position 1393, délétion d'un triplet GAT en position 1390 L'insertion du fragment PCR portant les séquences L2 dans le vecteur M13TG6131 conduit à M13TG9126. On dénombre 5 mutations ponctuelles par rapport à la séquence divulguée dans Genebank : C à la place d'un T en position 378, A à la place d'un G en position 691, A à la place d'un G en position 702, G à la place d'un A en position 990 et C à la place d'un A en position 1092. A titre indicatif, le vecteur M13TG6131 dérive de M13TG131 (Kieny et al., 1983, *supra)* par mutation du site *Bg*/II interne situé en dehors des sites multiples de clonage.

### B. Construction du vecteur pTG5021 pour le transfert des gènes L 1 et L2 dans le locus TK du génome du virus de la vaccine

Le gêne codant pour la protéine L1 est modifié par création d'un site *Bg*/II en amont de l'ATG initiateur. Le gène muté est excisé du vecteur précédent (M 13TG8185) par digestion *Bg*/II-*Sac*I et inséré entre les sites *Bam*HI et Sad du pTG186-poly. La construction résultante est dénommée pTG4019. Le vecteur de transfert pTG186-poly est décrit en détail dans le brevet français 2 583 429. Il comporte 10 sites de restriction pour l'insertion du gène à transférer et le promoteur p7,5K pour le contrôle de son expression.

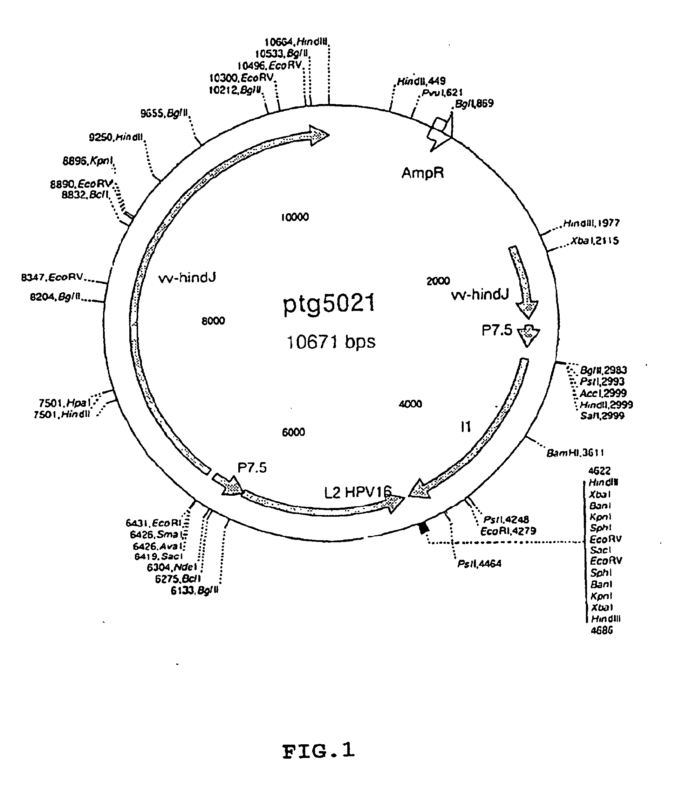

Le gène codant pour la protéine L2 est isolée de M13TG9126 par digestion *Bgl*II*-Hmd*III puis cloné entre les sites *Bam*HI et *Hm*dIII en 3' du promoteur 7,5K On obtient M13TG9127 dans lequel on introduit par mutagénèse localisée un site *Sal*I en amont du promoteur. Le bloc d'expression "p7,5K-gène L2" est isolé du vecteur muté M13TG9129 et cloné dans le site *Sac*I du vecteur de transfert pTG4019 de telle manière que les deux cassettes p7,5K-L1 et p7,5K-L2 soient en orientation inverse. La construction ainsi obtenue pTG5021 est représentée à la Figure 1. Les blocs d'expression sont transférés dans le génome du virus de la vaccine souche Copenhague par recombinaison homologue. Le virus recombinant désigné VVTG5021 est isolé par sélection au 5-bromodéoxyuridine (5BUDR)

### C. Isolement des gènes E6 et E7 de HPV16

Les gènes E6 et E7 sont isolés à partir de la lignée cellulaire Caski comme décrit aux exemples 2 et 3 du brevet européen EP 0 462 187. Deux constructions ont été dérivées du clone M13E7/E6 contenant les gènes E6 et E7 du HPV-16 afin de faciliter les étapes ultérieures de clonage. La première désignée M13TG8188 résulte de l'introduction par mutagénèse dirigée de sites *Pst*I et *Bam*HI respectivement en amont et en aval du gène E7 et la seconde, M13TG8189 comporte un site *Pst*I en amont du gène E6. L'introduction de mutations ponctuelles en amont d'un ATG initiateur et en aval d'un codon stop sont à la portée de l'homme de l'art

L'association de la protéine E7 de HPV-16 avec le produit du gène de rétinoblastome a été démontrée par divers auteurs (voir par exemple Munger et al., 1989, EMBO J. 8, 4099-4105) et corrélée à son pouvoir transformant. Pour des raisons évidentes de sécurité, on génère un mutant non oncogène délété des séquences codant pour les acides aminés 21 à 26 de la protéine E7 native impliqués dans la fonction de transformation par mutagénèse dirigée du vecteur M 13TG8188 à l'aide de l'oligonucléotide oTG5118 (SEQ ID NO. 1) On obtient M13TG9104. Le gène E7 muté est désigné ci-après E7*.

De même, il a été démontré que la protéine E6 de HPV-16 pouvait interagir avec le produit d'expression du gène suppresseur de tumeur *p53* (Crook et al., 1991, Cell 67, 547-556). Le domaine impliqué dans cette interaction a clairement été défini et se situe entre les résidus 111 à 115 de la protéine native. Le vecteur M13TG9125 est généré par mutagénèse de M 13TG8189 à l'aide de l'oligonucléotide oTG5377 (SEQ ID NO: 2). Le gène E6 muté est désigné ci-après E6*.

### D. Construction du vecteur de transfert pTG5065 porteur des gènes E6 et E7 de HPV-16 et du gène de l'IL-2 humaine intégrés au locus K1L.

Le fragment *Eco*RI K de 5,2 kb de l'extrémité gauche du génome du virus vaccine Copenhague (Guillard et al., 1985, J. Virl. *53,* 316-318) est cloné dans le plasmide pUC8 (Gibco BRL) linéarisé par *Eco*RI. Le vecteur ainsi obtenu est ensuite soumis à une digestion ménagée par *Bgl*II suivi d'une ligation afin de déléter un fragment de 855 pb codant pour le gène de restriction d'hôte K1 L (Guillard et al., 1986, Proc. Natl. Acad. Sci., USA 83, 5573-5577). Le fragment *Bgl*II de 3,4 kb est isolé de cette construction intermédiaire désignée pUC8Khr puis cloné dans le site *Bam*HI du plasmide pUC7 (Gibco BRL). On génère pBAC1 dans lequel on introduit un adaptateur *Xho*I à gauche du site *Bgl*II unique. Après digestion par *Xhol* et *Bgl*II, on insère un fragment *Sal*I*-Bgl*II portant le promoteur vaccine p7,5K. Les deux sites *Eco*RI sont éliminés par digestion *Eco*RI suivi d'un traitement par la Klenow et d'une religation. Le vecteur pTG2147 est obtenu par introduction dans le site unique *Bgl*II d'un site multiple de clonage isolé du vecteur p poly II (Lathe et al., *187,* Gene *57,* 193-201 ) isolé sous forme d'un fragment *Bgl*II*-Bam*HI II comprend donc les bras de recombinaison permettant l'insertion au locus K1L encadrant le promoteur p7,5K suivi des sites de restriction.

Les gènes E6* et E7* sont clonés en aval du promoteur vaccine pH5R contenu dans le vecteur M13TG9132, pour donner respectivement M 13TG9138 et M13TG9139. A titre indicatif, le vecteur M13TG9132 provient de l'insertion du promoteur du gène HSR isolé par PCR du génome viral dans le phage M13TG6131.

Par ailleurs, l'ADNc codant pour l'interleukine-2 humaine est isolé du plasmide pTG36 (brevet français 2 583 770), introduit dans un vecteur intermédiaire et resorti par digestion *Sal*I-*Bam*HI pour être inséré dans le vecteur de transfert pTG2147 ciblant le locus K1 L. L'insertion se fait au niveau des sites *Pst*I et *Xba*I situés en aval du promoteur p7,5K, à l'aide d'adaptateurs de clonage. On obtient pTG5056.

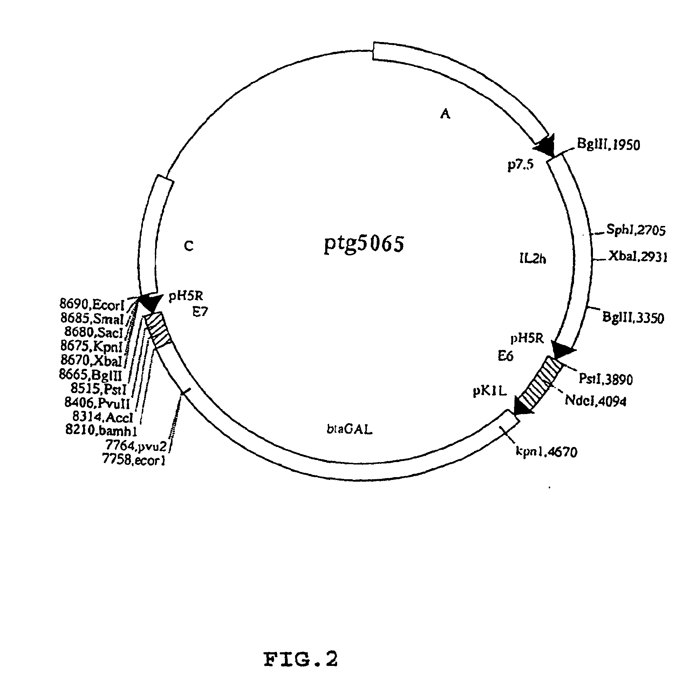

Le bloc d'expression pH5R-E6* est isolé du vecteur M13TG9139 sous forme d'un fragment *Bgl*II*-Bam*HI qui est introduit dans le site *Bam*HI du vecteur pTG5056 On génère le vecteur pTG5060. Le bloc d'expression pH5R-E7* est purifié de M13TG9138 et inséré au site *Bam*HI du vecteur pTG5060, pour donner pTG5062 On indique que l'insertion au locus K1L conduit à des virus recombinants ayant une capacité de réplication réduite voire même détruite dans certains types cellulaires. Pour ces raisons, on introduit un marqueur de sélection positive pour faciliter l'identification des virus recombinants. Le vecteur pTG5065 résulte du clonage dans le site *Bam*HI de pTG5062 d'une cassette d'expression "pK1 L-gène LacZ" isolée du plasmide pIV75 (Chen et al., 1993, Virology *196,* 682-693) par clivage *Bgl*II-*Bam*HI. Comme visualisé à la Figure 2, il permet le transfert des blocs E6*, E7* et IL-2 au sein du locus K1L du virus de la vaccine

Le virus de la vaccine recombinant, dénommé VVTG5065, est généré par recombinaison homologue après transfection du vecteur pTG5065 dans les cellules embryonnaires de poulet infectées par la vaccine Copenhague sauvage et repérées par coloration sous gélose au X-Gal. Les virus de la vaccine VVTG5021 et VVTG5065 sont utilisés dans des essais d'infections doubles Les doubles recombinants sont sélectionnés selon les deux marqueurs formation de plages bleues en présence de X-Gal et délétion du marqueur TK. Ceux-ci, dénommés VVTG5021 &5065, expriment les blocs p7,5K-IL-2, pH5R-E6* et pH5R-E7* intégrés au locus K1L et les blocs p7,5K-L1 et p7,5K-L2 intégrés au locus TK

L'expression des gènes de HPV peut être vérifiée par analyse en Western blot à l'aide d'anticorps monoclonaux ou polyclonaux appropriés ou par PCR réverse. La production d'IL-2 peut être évaluée par test ELISA ou test CTL comme décrit dans la littérature. A titre indicatif, le niveau d'expression in vitro est de l'ordre de 60 ng/ml/10⁶ cellules infectées à 1 pfu/cellule et 24h.

### EXEMPLE 2. Construction d'un virus vaccine Copenhague exprimant les gènes E6, E7, et le gène IL-2 humain

L'ADNc codant pour l'interleukine-2 humaine est isolé du plasmide pTG36 par digestion *Pst*I et inséré dans le site *Pst*I du plasmide pTG 186, donnant lieu à pTG188. Le virus obtenu par recombinaison homologue est dénommé VVTG188.

Le gène E6* est isolé de M13TG9125 sous forme d'un fragment *Pst*I-*Bam*HI et inséré en aval du promoteur 7,5K entre les sites *Pst*I et *Xbal* de pTG2147 en présence d'un adaptateur *Bam*HI-*Xba*I, donnant lieu à pTG5057. Le gène E7* est placé sous le contrôle du promoteur vaccine H5R produisant M13TG9138 et la cassette bordée par les sites *Bam*HI-*Bgl*II est sous-clonée dans le site *Bam*HI de pTG5057. On obtient pTG5059, dans le site *Bam*HI duquel on insère le marqueur de sélection *LacZ* sous la dépendance du promoteur du gène vaccine K1L isolé par digestion *Bgl*II*-Bam*HI du vecteur pIV75. La construction résultante est désignée pTG5061 et le virus généré par recombinaison homologue avec le génome vaccine VVTG5061.

Un virus de la vaccine recombinant exprimant les gènes E6* et E7* intégrés au locus K1L et le gène de l'IL-2 humaine intégré au locus TK est obtenu par co-infection de cellules embryonnaires de poulet par les virus VVTG5061 et VVTG188. A titre indicatif, ce dernier produit environ une quantité d'IL-2 supérieure à 400 ng pour 10⁶ cellules infectées à 1 pfu par cellule pendant 24h. Le virus doublement recombinant est désigné VVTG5061 &188.

### EXEMPLE 3: Construction d'un virus vaccine Copenhague exprimant les gènes E6, E7, et le gène codant pour le cofacteur d'adhésion B7.1 humain

L'ADNc codant pour B7.1 est isolé d'une préparation d'ARNm obtenue de la lignée cellulaire Daudi (ATCC CCL-213) par PCR réverse en utilisant les amorces oTG6353 et oTG6352 (SEQ ID N0: 3 et 4). On indique que la séquence du gène est divulguée dans Genebank sous le numéro d'accession M27533. Le fragment amplifié est cloné entre les sites *Bgl*II et *Eco*RI du M13TG6131 conduisant au M13TG9149 puis entre les sites *Bam*HI et *Eco*RI du pTG186. La construction est dénommée pTG5090 et le virus obtenu par recombinaison homologue VVTG5090. Un virus de la vaccine exprimant les gènes E6* et E7* de HPV-16 intégrés au locus K1L et le gène B7.1 humain intégré au locus TK peut être obtenu par co-infection de cellules embryonnaires de poulet par les virus VVTG5061 et VVTG5090. le virus recombinant est désigné VVTG5061 &5090.

### EXEMPLE 4 Construction d'un virus vaccine souche MVA exprimant les gènes E6, E7, et le gène B7 1 intégrés au sein de la zone d'excision III

Le virus MVA dérive de la souche du virus de la vaccine Ankara. Il n'est pas capable de générer des particules infectieuses sur les cellules de mammifères mais se développe correctement sur des fibroblastes embryonnaires de poulet. Son adaptation à ces cellules a provoqué l'excision de 6 régions non essentielles pour son développement et son cycle infectieux sur ce type de cellules (disparition d'environ 15 % du génome viral ; Meyer et al., 1991, J. Gen. Virol. 72, 1031-1038) L'intégration de matériel génétique exogène peut être réalisé au niveau de l'une quelconque de ces zones d'excision. Dans le cadre de la présente invention, on utilise les excisions II et III localisées au niveau des fragments de restriction *Hin*dIII N et A respectivement (Altenburger et al., 1989, Arch. Virol. *105*, 15-27)

Dans un premier temps, on construit le vecteur pTG6019 permettant l'insertion dans la zone d'excision III du virus MVA. Les bras de recombinaison homologue de part et d'autre de la zone d'excision III sont isolés par PCR à partir du génome viral (voir brevet américain US 5,185,146) et des amorces oTG7637 et oTG7638 (SEQ ID NO: 5 et 6) pour le bras gauche et oTG7635 et oTG7636 (SEQ ID NO 7 et 8) pour le bras droit. Les fragments amplifiés sont clonés au site *Eco*RI du vecteur pTG1E, pour donner pTG6019. Le matériel génétique à transférer est inséré entre les deux bras de recombinaison. Le vecteur pTG1E est similaire au pTGIH (brevet français 2 583 429) mis à part la présence d'un adaptateur *Eco*RI à la place de sites multiples de clonage.

On insère en premier lieu une cassette d'expression du gène marqueur *gus A* Le promoteur 7,5K est tout d'abord cloné dans le site *Bam*HI de pTG6019 On obtient pTG6021 dans le site *Bam*HI duquel on insère le gène *gus* A généré sous forme d'un fragment *Bgl*II*-Bam*HI. Celui-ci peut être obtenu à partir de la séquence divulguée dans la littérature La construction résultante est dénommée pTG6022. La présence du marqueur va permettre de discriminer les virus sauvages des virus recombinants par détection de l'activité enzymatique GUS par le substrat XglcA. Une coloration rouge révèle l'activité β-glucuronidase. Cependant, dans l'optique d'une application clinique, il peut être utile d'être en mesure d'éliminer ce marqueur bactérien du produit final après la sélection des virus recombinants. Pour ce faire, on met à profit la capacité de la vaccine à déléter les séquences comprises entre deux sites homologues C'est pourquoi on insère un second promoteur p7,5K en aval du gène *gus A* dans une orientation sens par rapport à celui qui dirige l'expression de ce dernier. Le vecteur pTG6022 est modifié par insertion entre les sites *Bam*HI et *Sac*I d'un fragment p7,5K muni d'extrémités cohésives, pour donner pTG6025.

Celui-ci est complété par l'insertion des cassettes d'expression des gènes E6* et E7* mutées. On commence par introduire une nouvelle séquence promotrice p7,5K cette fois en orientation antisens par rapport aux précédentes Cette construction dénommée pTG6039 comprend donc la cassette GUS labile "p7,5K→*gus A*-p7,5K→" suivie de p7,5K dans l'orientation opposée. En parallèle, les gènes E6* et E7* sont isolés respectivement des vecteurs M13TG9104 et M13TG9125 par digestion *Bam*HI et *Pst*I et assemblés en orientation opposée l'un à l'autre au site *Pst*I de M13TG6131. L'ensemble est resorti sous forme d'un fragment *Pst*I qui est inséré dans pTG6039 linéarisé par cette même enzyme. On obtient le vecteur pTG6056 qui contient les séquences suivantes p7,5K→*gus A*-p7,5K→E7*-E6*←p7,5K".

Le gène immunostimulateur B7.1 est intégré dans cette dernière construction. Pour ce faire, le vecteur pTG6056 est clivé par *Hind*III et *Kpn*l avant d'être mis en ligation en présence des oligonucléotides oTG10451 et oTG10450 (SEQ ID NO: 9 et 10), pour générer pTG6070. Ces derniers vont permettre le clonage de la cassette d'expression "pH5R-B7.1" par recombinaison homologue. A cet effet, les séquences B7.1 isolées de M13TG9149 sont clonées en aval du promoteur vaccine pH5R, pour former M13TG9184. Le fragment *Bgl*II*-Eco*RI portant la cassette est intégré dans le vecteur pTG6070 linéarisé par *Xhol* par recombinaison homologue dans *E. coli*. On obtient le vecteur pTG6079 qui au total comporte le gêne marqueur *gus A* sous une forme "labile" et les cassettes d'expression des gènes précoces de HPV-16 ainsi qu'une cassette du gène de costimulation B7.1. Le virus généré par recombinaison homologue avec le génome MVA est désigné MVATG6079.

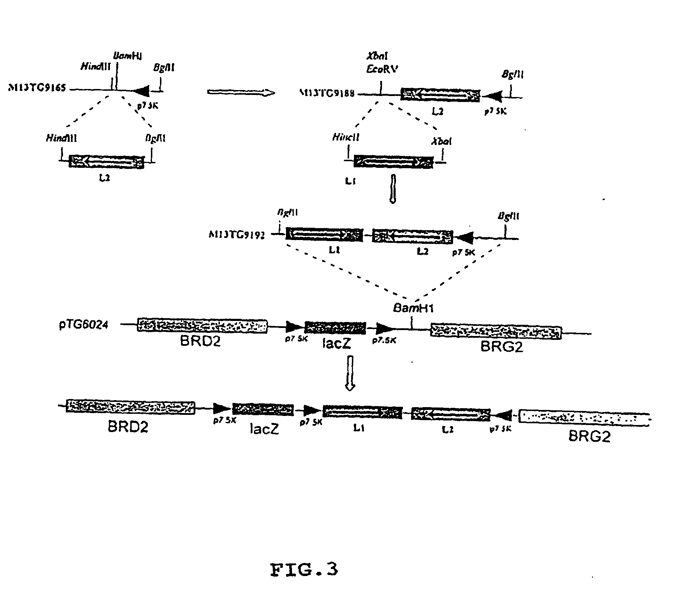

Les blocs d'expression des gènes tardifs du HPV-16 peuvent être insérés au sein de la zone d'excision II à l'aide du vecteur de transfert pTG6018. Celui-ci est construit par insertion au site *Eco*RI de pTG1E des bras de recombinaison gauche et droit bordant l'excision II, générés par PCR en utilisant les amorces oTG7665 et oTG7584 (SEQ ID NO: 11 et 12) et oTG7639 et oTG7640 (SEQ ID N0 13 et 14) Comme précédemment, on intègre entre les deux bras une cassette d'expression d'un marqueur positif pour faciliter la détection des virus recombinants, celui-ci pouvant être éliminé après l'étape de sélection (Spehner et al, 1990, J Virol. *64,* 527-533). Le vecteur pTG6020 résulte du clonage du gène *LacZ* placé en aval du promoteur p7,5K dans le vecteur pTG6018 linéarisé par *Bam*HI. Le pTG6020 est modifié par l'insertion d'une séquence p7,5K entre ses sites *Bam*HI et *Sac*I situés en aval du gène *LacZ*. On obtient pTG6024. Les cassettes L 1 et L2 peuvent ensuite être introduites selon une stratégie telle que montrée à la Figure 3.

### EXEMPLE 5: Construction d'un virus vaccine souche MVA exprimant les gènes E6, E7, et le gène IL-2 intégrés au sein de la zone d'excision III

On utilise la même stratégie que précédemment pour introduire le gène IL-2 dans le vecteur pTG6056. Après clonage des oligonucléotides de recombinaison oTG10503 et oTG10502 (SEQ ID NO: 15 et 16) pour produire pTG6076, ce dernier est clivé par *Xho*I et le fragment *Bgl*II*-Eco*RI préparé à partir de M13TG9185 (pH5R-IL-2) inséré par recombinaison homologue. Le vecteur pTG6090 ainsi obtenu comprend le gène marqueur *gus A* sous une forme "labile", les cassettes d'expression des gènes précoces de HPV-16 ainsi qu'une cassette du gène IL-2 humain. Le virus recombinant obtenu par recombinaison homologue avec le génome MVA est désigné MVATG6090. Les gènes tardifs peuvent être intégrés dans la zone d'excision II en mettant en oeuvre le pTG6018, comme indiqué ci-dessus.

### EXEMPLE 6: Validation du vecteur VVTG5021 &5065 en modèle animal

### A. Etudes de toxicité

Des souris nude ont reçu 10⁷ pfu de VVTG5021 &5065 ou 10⁷ pfu de virus vaccine sauvage par voie intraveineuse, intramusculaire, sous-cutanée ou intracraniale. Des lots de 5 souris sont constitués selon le type de virus injecté et la voie d'administration et on évalue 26 jours après l'injection, le nombre d'animaux présentant des lésions liées à la vaccine. Les souris ayant reçu le virus recombinant ne montrent aucune lésion quelles que soient la voie d'administration utilisée alors que la majorité des animaux traités avec la vaccine sauvage présente des lésions avec une fréquence et une gravité fonction de la voie d'administration. De plus, des décès sont enregistrés dans le cas d'une injection intraveineuse et intracraniale. Ces données montrent que le VVTG5021 &5065 est atténué par rapport au virus sauvage et qu'il n'entraîne pas de lésions même après une injection intracraniale.

### B. Expériences d'immunoprophylaxie avec le VVTG5021&5065

Des souris C57BL6 ont été vaccinées à trois reprises par voie sous-cutanée avec 10⁷ pfu de VVTG5021 &5065. Trois jours après la dernière immunisation, ces animaux sont éprouvés avec 10³ cellules E7W1 implantées en sous-cutané. A titre indicatif, les cellules E7W1 proviennent d'une lignée de lymphome murin transfectée par un vecteur exprimant le gène E7 oncogéne de HPV-16. Le pourcentage de survie des animaux en fonction du temps est comparé à celui obtenu avec des souris témoins traitées avec 10⁷ pfu d'un virus de la vaccine non recombinant VVTG186 (dérivé du vecteur TG186 décrit ci-dessus). Le suivi de la mortalité montre une différence entre les deux groupes. Dans le groupe témoin, 100 % des animaux sont morts à J36 alors que 40 % des animaux vaccinés par VVTG5021 &5065 sont encore vivants à J36 et plus de 30 % à J51.

Ainsi les virus recombinants exprimant des antigènes de HPV et un gène immunostimulateur présentent une activité antitumorale à l'encontre des cellules tumorales exprimant l'oncogène E7 de H PV-16.

### C. Expériences d'immunothérapie

Des souris C57 BL6 sont inoculées avec 10³ cellules E7 WI inplantées en sous-cutanée (J0). 10⁷ pfu de virus recombinants, sont ensuite administrés également en sous-cutanée à J3 puis J6 et enfin J9 et le pourcentage de survie des animaux est déterminé par rapport aux animaux contrôles ayant reçu un virus non recombinant. Alors que 100 % des animaux contrôles sont morts, on observe un accroissement notable de la survie des animaux injectés avec un mélange de VVTG5061§5021 et de VVTG188. Des résultats similaires sont obtenus après administration de VVTG5065§5021 et VVTG188.

Ces expériences d'immunothérapie ont été reproduites en utilisant un modèle tumoral différent, des cellules BMK16 myc remplaçant les cellules E7w1. Les cellules BMK16myc sont des cellules de rein de souris nouveau-nées transfectées par le génome de HPV-16 et le gène c myc murin. Les animaux sont traités par 10⁷ virus MVA TG6090 exprimant les gènes E6*, E7* de HPV-16 et l'IL-2 humaine. Par rapport aux contrôles, les souris traitées présentent un retard de la croissance tumorale jusqu'à J15.

## Revendications

1. Composition pharmaceutique destinée au traitement ou à la prévention d'une infection ou tumeur à papillomavirus qui comprend à titre d'agents thérapeutiques un polypeptide originaire de la région précoce E6, un polypeptide originaire de la région précoce E7, un polypeptide originaire de la région tardive L1, un polypeptide originaire de la région tardive L2 d'un papillomavirus et au moins un polypeptide ayant une activité immunostimulatrice sélectionné parmi le groupe constitué par l'interleukine-2, l'interleukine-7 et les molécules de co-adhésion B7.1 et B7.2;
étant entendu que ledit polypeptide ayant une activité immunostimulatrice est un polypeptide natif et lesdits polypeptides originaires de la région précoce et lesdits polypeptides originaires de la région tardive sont des polypeptides natifs ou des variants **caractérisés par** au moins une mutation d'un acide aminé.

2. Composition pharmaceutique selon la revendication 1, **caractérisée en ce que** le polypeptide originaire de la région précoce d'un papillomavirus est un variant non oncogène de la protéine E6 et/ou E7 d'un papillomavirus.

3. Composition pharmaceutique selon la revendication 1, **caractérisée en ce que** le polypeptide ayant une activité immunostimulatrice dérive de l'interleukine-2.

4. Composition pharmaceutique selon la revendication 1, **caractérisée en ce que** le polypeptide ayant une activité immunostimulatrice dérive de la molécule B7.1.

5. Composition pharmaceutique selon l'une des revendications 1 à 4, **caractérisée en ce qu'**elle comprend :
(1) un polypeptide originaire de la région E6, un polypeptide originaire de la région E7, un polypeptide originaire de la région L1, un polypeptide originaire de la région L2 d'un papillomavirus et un polypeptide dérivé de l'interleukine-2,
(2) un polypeptide originaire de la région E6, un polypeptide originaire de la région E7, un polypeptide originaire de la région L1, un polypeptide originaire de la région L2 d'un papillomavirus et un polypeptide dérivé de la molécule B7.1, ou
(3) un polypeptide originaire de la région E6, un polypeptide originaire de la région E7, un polypeptide originaire de la région L1, un polypeptide originaire de la région L2 d'un papillomavirus, un polypeptide dérivé de la molécule B7.1 et un polypeptide dérivé de l'interleukine-2.

6. Composition pharmaceutique selon l'une des revendications 1 à 5, **caractérisée en ce que** le papillomavirus est sélectionné parmi les type HPV-16, HPV-18, HPV-31, HPV-33 et/ou HPV-45.

7. Composition pharmaceutique destinée au traitement ou à la prévention d'une infection ou tumeur à papillomavirus qui comprend à titre d'agent(s) thérapeutique(s) un ou plusieurs vecteur(s) recombinant(s) dérivant d'un poxvirus dans le(s)quel(s) sont insérés des fragments d'ADN codant pour :
(1) un polypeptide originaire de la région précoce E6, un polypeptide originaire de la région précoce E7, un polypeptide originaire de la région L1, un polypeptide originaire de la région L2 d'un papillomavirus, ou
(2) au moins un polypeptide originaire de la région précoce d'un papillomavirus, au moins un polypeptide originaire de la région tardive d'un papillomavirus et au moins un polypeptide ayant une activité immunostimulatrice sélectionné parmi le groupe constitué par l'interleukine-2, l'interleukine-7 et les molécules de co-adhésion B7.1 et B7.2,
lesdits fragments d'ADN étant placés sous le contrôle des éléments indépendants nécessaires à leur expression dans une cellule ou un organisme hôte.

8. Composition pharmaceutique selon la revendication 7, **caractérisée en ce que** lesdits polypeptides ont les caractéristiques définies aux revendications 2 à 6.

9. Composition pharmaceutique selon l'une des revendications 7 et 8, **caractérisée en ce que** le poxvirus est sélectionné parmi le groupe constitué par le virus de la vaccine, le canaripox et le fowlpox.

10. Composition pharmaceutique selon la revendication 9, **caractérisée en ce que** le vecteur recombinant dérive d'un virus de la vaccine sélectionné parmi les souches Copenhague, Wyeth et Ankara modifiée (MVA).

11. Composition pharmaceutique selon l'une des revendications 7 à 10, **caractérisée en ce que** les éléments essentiels à l'expression des fragments d'ADN codant pour lesdits polypeptides comprennent un promoteur d'un gène d'un virus de la vaccine sélectionné parmi les promoteurs des gènes thymidine kinase (TK), 7.5K, H5R et KIL.

12. Composition pharmaceutique selon la revendication 10 ou 11, **caractérisée en** e que le vecteur recombinant dérive d'un virus de la vaccine de la souche Copenhague et que les fragments d'ADN codant pour lesdits polypeptides sont insérés dans le locus TK et/ou le locus KIL dudit virus de la vaccine.

13. Composition pharmaceutique selon la revendication 10 ou 11, **caractérisée en ce que** le vecteur recombinant dérive d'un virus de la vaccine de la souche MVA et que les fragments d'ADN codant pour lesdits polypeptides sont insérés au niveau de l'une quelconque des zones d'excision sélectionnées parmi les excisions I, II, III, IV, V et VI dudit virus de la vaccine.

14. Composition pharmaceutique selon l'une des revendications 7 à 13, destinée au traitement ou la prévention d'une infection ou tumeur à papillomavirus, **caractérisée en ce qu'**elle comprend un ou plusieurs vecteur(s) recombinant(s) dérivé(s) d'un virus de la vaccine dans le(s)quel(s) sont insérés :
(1) au moins un fragment d'ADN codant pour au moins un polypeptide originaire de la région tardive d'un papillomavirus et un fragment d'ADN codant pour la protéine E6 d'un papillomavirus, un fragment d'ADN codant pour la protéine E7 d'un papillomavirus et un fragment d'ADN codant pour la molécule B7.1,
(2) au moins un fragment d'ADN codant pour au moins un polypeptide originaire de la région tardive d'un papillomavirus et un fragment d'ADN codant pour la protéine E6 d'un papillomavirus, un fragment d'ADN codant pour la protéine E7 d'un papillomavirus et un fragment d'ADN codant pour l'interleukine-2,
(3) au moins un fragment d'ADN codant pour au moins un polypeptide originaire de la région tardive d'un papillomavirus et un fragment d'ADN codant pour la protéine E6 d'un papillomavirus, un fragment d'ADN codant pour la protéine E7 d'un papillomavirus, un fragment d'ADN codant pour la molécule B7.1 et un fragment d'ADN codant pour l'interleukine-2,
(4) un fragment d'ADN codant pour la protéine E6 d'un papillomavirus, un fragment d'ADN codant pour la protéine E7 d'un papillomavirus, un fragment d'ADN codant pour la protéine L1 d'un papillomavirus, un fragment d'ADN codant pour la protéine L2 d'un papillomavirus et un fragment d'ADN codant pour la molécule B7.1,
(5) un fragment d'ADN codant pour la protéine E6 d'un papillomavirus, un fragment d'ADN codant pour la protéine E7 d'un papillomavirus, un fragment d'ADN codant pour la protéine L1 d'un papillomavirus, un fragment d'ADN codant pour la protéine L2 d'un papillomavirus et un fragment d'ADN codant pour l'interleukine-2, ou
(6) un fragment d'ADN codant pour la protéine E6 d'un papillomavirus, un fragment d'ADN codant pour la protéine E7 d'un papillomavirus, un fragment d'ADN codant pour la protéine L1 d'un papillomavirus, un fragment d'ADN codant pour la protéine L2 d'un papillomavirus, un fragment d'ADN codant pour la molécule B7.1 et un fragment d'ADN codant pour l'interleukine-2.

15. Composition pharmaceutique selon la revendication 7, **caractérisée en ce qu'**elle comprend, à titre d'agents thérapeutiques, un premier vecteur recombinant dans lequel est(sont) inséré(s) un ou plusieurs fragments d'ADN codant pour les(s)dit(s) polypeptide(s) originaire(s) de la région précoce d'un papillomavirus et un ou plusieurs fragments d'ADN codant pour le(s)dit(s) polypeptide(s) originaire(s) de la région tardive d'un papillomavirus et un second vecteur recombinant dans lequel est (sont) inséré(s) un ou plusieurs fragment(s) d'ADN codant pour ledit polypeptide ayant une activité immunostimulatrice.

16. Composition pharmaceutique selon la revendication 15, **caractérisée en ce que** les fragments d'ADN insérés dans le premier vecteur codent pour E6, E7, L1 et L2 et **en ce que** le fragment d'ADN inséré dans le second vecteur code pour IL-2.

17. Composition pharmaceutique selon l'une des revendications 1 à 16, **caractérisée en ce qu'**elle comporte un support acceptable d'un point de vue pharmaceutique, permettant son administration par injection à l'homme ou à l'animal.

18. Composition pharmaceutique selon l'une des revendications 1 à 17, à titre de médicament pour le traitement ou la prévention du cancer du col de l'utérus, d'une dysplasie du col de bas grade et d'une infection à papillomavirus.

## Patentansprüche

1. Pharmazeutische Zusammensetzung zur Behandlung oder Vorbeugung einer Infektion oder eines Tumors, verursacht durch ein Papillomavirus, welche als therapeutische Wirkstoffe ein Polypeptid, welches aus der frühen Region E6 stammt, ein Polypeptid, welches aus der frühen Region E7 stammt, ein Polypeptid, welches aus der späten Region L1 stammt, ein Polypeptid, welches aus der späten Region L2 eines Papillomavirus stammt, und wenigstens ein Polypeptid mit immunstimulierender Wirkung, ausgewählt aus der Gruppe Interleukin-2, Interleukin-7 und den Coadhäsionsmolekülen B7.1 und B7.2, enthält,
wobei das Polypeptid mit immunstimulierender Wirkung ein natives Polypeptid ist, und die Polypeptide, die aus der frühen Region stammen, und die Polypeptide, die aus der späten Region stammen, native Proteine oder Varianten, **gekennzeichnet durch** wenigstens eine Mutation einer Aminosäure, sind.

2. Pharmazeutische Zusammensetzung gemäß Anspruch 1, **dadurch gekennzeichnet, dass** das Polypeptid, welches aus der frühen Region eines Papillomavirus stammt, eine nicht onkogene Variante des Proteins E6 und/oder E7 eines Papillomavirus ist.

3. Pharmazeutische Zusammensetzung gemäß Anspruch 1, **dadurch gekennzeichnet, dass** das Polypeptid mit immunstimulierender Wirkung vom Interleukin-2 abgeleitet ist.

4. Pharmazeutische Zusammensetzung gemäß Anspruch 1, **dadurch gekennzeichnet, dass** das Polypeptid mit immunstimulierender Wirkung vom Molekül B7.1 abgeleitet ist.

5. Pharmazeutische Zusammensetzung gemäß einem der Ansprüche 1 bis 4, **dadurch gekennzeichnet, dass** sie:
(1) ein Polypeptid, welches aus der Region E6 stammt, ein Polypeptid, welches aus der Region E7 stammt, ein Polypeptid, welches aus der Region L1 stammt, ein Polypeptid, welches aus der Region L2 eines Papillomavirus stammt, und ein Polypeptid, welches vom Interleukin-2 abgeleitet ist,
(2) ein Polypeptid, welches aus der Region E6 stammt, ein Polypeptid, welches aus der Region E7 stammt, ein Polypeptid, welches aus der Region L1 stammt, ein Polypeptid, welches aus der Region L2 eines Papillomavirus stammt, und ein Polypeptid, welches vom Molekül B7.1 abgeleitet ist, oder
(3) ein Polypeptid, welches aus der Region E6 stammt, ein Polypeptid, welches aus der Region E7 stammt, ein Polypeptid, welches aus der Region L1 stammt, ein Polypeptid, welches aus der Region L2 eines Papillomavirus stammt, ein Polypeptid, welches vom Molekül B7.1 abgeleitet ist, und ein Polypeptid, welches vom Interleukin-2 abgeleitet ist, enthält.

6. Pharmazeutische Zusammensetzung gemäß einem der Ansprüche 1 bis 5, **dadurch gekennzeichnet, dass** das Papillomavirus unter den Typen HPV-16, HPV-18, HPV-31, HPV-33 und/oder HPV-45 ausgewählt ist.

7. Pharmazeutische Zusammensetzung zur Behandlung oder Vorbeugung einer Infektion oder eines Tumors, verursacht durch ein Papillomavirus, welche als therapeutische(n) Wirkstoff(e) einen oder mehrere rekombinante Vektoren enthält, der/die von einem Pockenvirus abgeleitet ist/sind, in den/die DNA-Fragmente eingefügt sind, welche:
(1) ein Polypeptid, welches aus der frühen Region E6 stammt, ein Polypeptid, welches aus der frühen Region E7 stammt, ein Polypeptid, welches aus der Region L1 stammt, und ein Polypeptid, welches aus der Region L2 eines Papillomavirus stammt, oder
(2) wenigstens ein Polypeptid, welches aus der frühen Region eines Papillomavirus stammt, wenigstens ein Polypeptid, welches aus der späten Region eines Papillomavirus stammt, und wenigstens ein Polypeptid mit immunstimulierender Wirkung, ausgewählt aus der Gruppe Interleukin-2, Interleukin-7 und den Coadhäsionsmolekülen B7.1 und B7.2, kodieren,
wobei die DNA-Fragmente unter der Kontrolle von unabhängigen Elementen platziert sind, die für ihre Expression in einer Wirtszelle oder einem Wirtsorganismus notwendig sind.

8. Pharmazeutische Zusammensetzung gemäß Anspruch 7, **dadurch gekennzeichnet, dass** die Polypeptide die in den Ansprüchen 2 bis 6 definierten Eigenschaften aufweisen.

9. Pharmazeutischen Zusammensetzung gemäß einem der Ansprüche 7 und 8, **dadurch gekennzeichnet, dass** das Pockenvirus ausgewählt ist aus der Gruppe Kuhpockenvirus, Canaripockenvirus und Geflügelpockenvirus.

10. Pharmazeutische Zusammensetzung gemäß Anspruch 9, **dadurch gekennzeichnet, dass** der rekombinante Vektor von einem Kuhpockenvirus, ausgewählt aus den Stämmen Kopenhagen, Wyeth und modifizierter Ankara (MVA), abgeleitet ist.

11. Pharmazeutische Zusammensetzung gemäß einem der Ansprüche 7 bis 10, **dadurch gekennzeichnet, dass** die Elemente, die für die Expression der die Polypeptide kodierenden DNA-Fragmente notwendig sind, einen Promotor eines Kuhpockenvirusgens, ausgewählt aus den Promotoren der Thymidinkinase(TK)-, 7.5K-, H5R- und KIL-Gene, umfassen.

12. Pharmazeutische Zusammensetzung gemäß Anspruch 10 oder 11, **dadurch gekennzeichnet, dass** der rekombinante Vektor von einem Kuhpockenvirus des Stammes Kopenhagen abgeleitet ist, und dass die DNA-Fragmente, welche die Polypeptide kodieren, in den TK-Lokus und/oder den KIL-Lokus des Kuhpockenvirus eingefügt sind.

13. Pharmazeutische Zusammensetzung gemäß Anspruch 10 oder 11, **dadurch gekennzeichnet, dass** der rekombinante Vektor von einem Kuhpockenvirus des Stammes MVA abgeleitet ist, und dass die DNA-Fragmente, welche die Polypeptide kodieren, auf Höhe irgend eines der Ausschnittsbereiche I, II, III, IV, V und/oder VI des Kuhpockenvirus eingefügt sind.

14. Pharmazeutische Zusammensetzung gemäß einem der Ansprüche 7 bis 13 zur Behandlung oder Vorbeugung einer Infektion oder eines Tumors, verursacht durch ein Papillomavirus, **dadurch gekennzeichnet, dass** sie einen oder mehrere rekombinante Vektoren enthält, der/die von einem Kuhpockenvirus abgeleitet ist/sind, in den/die:
(1) wenigstens ein DNA-Fragment, welches wenigstens ein Polypeptid kodiert, welches aus der späten Region eines Papillomavirus stammt, und ein DNA-Fragment, welches das Protein E6 eines Papillomavirus kodiert, ein DNA-Fragment, welches das Protein E7 eines Papillomavirus kodiert, und ein DNA-Fragment, welches das Molekül B7.1 kodiert,
(2) wenigstens ein DNA-Fragment, welches wenigstens ein Polypeptid kodiert, welches aus der späten Region eines Papillomavirus stammt, und ein DNA-Fragment, welches das Protein E6 eines Papillomavirus kodiert, ein DNA-Fragment, welches das Protein E7 eines Papillomavirus kodiert, und ein DNA-Fragment, welches das Interleukin-2 kodiert,
(3) wenigstens ein DNA-Fragment, welches wenigstens ein Polypeptid kodiert, welches aus der späten Region eines Papillomavirus stammt, und ein DNA-Fragment, welches das Protein E6 eines Papillomavirus kodiert, ein DNA-Fragment, welches das Protein E7 eines Papillomavirus kodiert, ein DNA-Fragment, welches das Molekül B7.1 kodiert, und ein DNA-Fragment, welches das Interleukin-2 kodiert,
(4) ein DNA-Fragment, welches das Protein E6 eines Papillomavirus kodiert, eine DNA-Fragment, welches das Protein E7 eines Papillomavirus kodiert, ein DNA-Fragment welches das Protein L1 eines Papillomavirus kodiert, ein DNA-Fragment, welches das Protein L2 eines Papillomavirus kodiert, und ein DNA-Fragment, welches das Molekül B7.1 kodiert,
(5) ein DNA-Fragment, welches das Protein E6 eines Papillomavirus kodiert, ein DNA-Fragment, welches das Protein E7 eines Papillomavirus kodiert, ein DNA-Fragment, welches das Protein L1 eines Papillomavirus kodiert, ein DNA-Fragment, welches das Protein L2 eines Papillomavirus kodiert, und ein DNA-Fragment, welches das Interleukin-2 kodiert, oder
(6) ein DNA-Fragment, welches das Protein E6 eines Papillomavirus kodiert, ein DNA-Fragment, welches das Protein E7 eines Papillomavirus kodiert, ein DNA-Fragment, welches das Protein L1 eines Papillomavirus kodiert, ein DNA-Fragment, welches das Protein L2 eines Papillomavirus kodiert, ein DNA-Fragment, welches das Molekül B7.1 kodiert, und ein DNA-Fragment, welches das Interleukin-2 kodiert,
eingefügt sind.

15. Pharmazeutische Zusammensetzung gemäß Anspruch 7, **dadurch gekennzeichnet, dass** sie als therapeutische Wirkstoffe einen ersten rekombinanten Vektor, in den ein oder mehrere DNA-Fragmente, die das/die Polypeptid(e) kodieren, welche(s) aus der frühen Region eines Papillomavirus stammt/stammen, und ein oder mehrere DNA-Fragmente, die das/die Polypeptid(e) kodieren, welche(s) aus der späten Region eines Papillomavirus stammt/stammen, eingefügt sind, und einen zweiten rekombinanten Vektor, in den ein oder mehrere DNA-Fragmente eingefügt sind, welche das Polypeptid mit immunstimulierender Wirkung kodieren, enthält.

16. Pharmazeutische Zusammensetzung gemäß Anspruch 15, **dadurch gekennzeichnet, dass** die DNA-Fragmente, die in den ersten Vektor eingefügt sind, E6, E7, L1 und L2 kodieren, und dass das DNA-Fragment, welches in den zweiten Vektor eingefügt ist, IL-2 kodiert.

17. Pharmazeutische Zusammensetzung gemäß einem der Ansprüche 1 bis 16, **dadurch gekennzeichnet, dass** sie einen in pharmazeutischer Hinsicht akzeptablen Träger enthält, der ihre Verabreichung an einen Menschen oder ein Tier durch Injektion ermöglicht.

18. Pharmazeutische Zusammensetzung gemäß einem der Ansprüche 1 bis 17 als Medikament zur Behandlung oder Vorbeugung von Gebärmutterhalskrebs, einer Dysplasie des Halses niederen Grads und einer Papillomavirus-Infektion.

## Claims

1. Pharmaceutical composition intended for the treatment or prevention of a papillomavirus infection or tumour, which comprises, as therapeutic agents, a polypeptide from the E6 early region, a polypeptide from the E7 early region, a polypeptide from the L1 late region, a polypeptide from the L2 late region of a papillomavirus and at least one polypeptide having immunostimulatory activity, selected from the group consisting of interleukin-2, interleukin-7 and co-adhesion molecules B7.1 and B7.2; it being understood that the said polypeptide having immunostimulatory activity is a native polypeptide and the said polypeptides from the early region and the said polypeptides from the late region are native polypeptides or variants **characterized by** at least one mutation of an amino acid.

2. Pharmaceutical composition according to Claim 1, **characterized in that** the polypeptide from the early region of a papillomavirus is a nononcogenic variant of the E6 and/or E7 protein of a papillomavirus.

3. Pharmaceutical composition according to Claim 1, **characterized in that** the polypeptide having immunostimulatory activity is derived from interleukin-2.

4. Pharmaceutical composition according to Claim 1, **characterized in that** the polypeptide having immunostimulatory activity is derived from the molecule B7.1.

5. Pharmaceutical composition according to one of Claims 1 to 4, **characterized in that** it comprises:
(1) a polypeptide from the E6 region, a polypeptide from the E7 region, a polypeptide from the L1 region, a polypeptide from the L2 region of a papillomavirus and a polypeptide derived from interleukin-2,
(2) a polypeptide from the E6 region, a polypeptide from the E7 region, a polypeptide from the L1 region, a polypeptide from the L2 region of a papillomavirus and a polypeptide derived from the molecule B7.1, or
(3) a polypeptide from the E6 region, a polypeptide from the E7 region, a polypeptide from the L1 region, a polypeptide from the L2 region of a papillomavirus, a polypeptide derived from the molecule B7.1 and a polypeptide derived from interleukin-2.

6. Pharmaceutical composition according to one of Claims 1 to 5, **characterized in that** the papillomavirus is selected from the HPV-16, HPV-18, HPV-31, HPV-33 and/or HPV-45 types.

7. Pharmaceutical composition intended for the treatment or prevention of a papillomavirus infection or tumour, which comprises, as therapeutic agent(s), one or more recombinant vector(s) derived from a poxvirus into which there are inserted DNA fragments coding for:
(1) a polypeptide from the E6 early region, a polypeptide from the E7 early region, a polypeptide from the L1 region, a polypeptide from the L2 region from a papillomavirus, or
(2) at least one polypeptide from the early region of a papillomavirus, at least one polypeptide from the late region of a papillomavirus and at least one polypeptide having immunostimulatory activity, selected from the group consisting of interleukin-2, interleukin-7 and co-adhesion molecules B7.1 and B7.2, the said DNA fragments being placed under the control of the elements necessary for their expression in a host cell or organism.

8. Pharmaceutical composition according to Claim 7, **characterized in that** the said polypeptides have the characteristics defined in Claims 2 to 6.

9. Pharmaceutical composition according to either of Claims 7 and 8, **characterized in that** the poxvirus is selected from the group consisting of vaccinia virus, canarypox virus and fowlpox virus.

10. Pharmaceutical composition according to Claim 9, **characterized in that** the recombinant vector is derived from a vaccinia virus selected from the Copenhagen, Wyeth and modified Ankara (MVA) strains.

11. Pharmaceutical composition according to any one of Claims 7 to 10, **characterized in that** the elements essential for the expression of the DNA fragments coding for the said polypeptides comprise a promoter of a gene of a vaccinia virus selected from the promoters of the thymidine kinase (TK), 7.5K, H5R and K1L genes.

12. Pharmaceutical composition according to Claim 10 or 11, **characterized in that** the recombinant vector is derived from a vaccinia virus of the Copenhagen strain and **in that** the DNA fragments coding for the said polypeptides are inserted into the TK locus and/or the K1L locus of the said vaccinia virus.

13. Pharmaceutical composition according to Claim 10 or 11, **characterized in that** the recombinant vector is derived from a vaccinia virus of the MVA strain and **in that** the DNA fragments coding for the said polypeptides are inserted at the level of any of the excision zones selected from the I, II, III, IV, V and VI excisions of the said vaccinia virus .

14. Pharmaceutical composition according to one of Claims 7 to 13, intended for the treatment or prevention of a papillomavirus infection or tumour, **characterized in that** it comprises one or more recombinant vectors derived from a vaccinia virus into which there are inserted:
(1) at least one DNA fragment coding for at least one polypeptide from the late region of a papillomavirus and a DNA fragment coding for the E6 protein of a papillomavirus, a DNA fragment coding for the E7 protein of a papillomavirus and a DNA fragment coding for the molecule B7.1,
(2) at least one DNA fragment coding for at least one polypeptide from the late region of a papillomavirus and a DNA fragment coding for the E6 protein of a papillomavirus, a DNA fragment coding for the E7 protein of a papillomavirus and a DNA fragment coding for interleukin-2,
(3) at least one DNA fragment coding for at least one polypeptide from the late region of a papillomavirus and a DNA fragment coding for the E6 protein of a papillomavirus, a DNA fragment coding for the E7 protein of a papillomavirus, a DNA fragment coding for the molecule B7.1 and a DNA fragment coding for interleukin-2,
(4) a DNA fragment coding for the E6 protein of a papillomavirus, a DNA fragment coding for the E7 protein of a papillomavirus, a DNA fragment coding for the L1 protein of a papillomavirus, a DNA fragment coding for the L2 protein of a papillomavirus and a DNA fragment coding for the molecule B7.1,
(5) a DNA fragment coding for the E6 protein of a papillomavirus, a DNA fragment coding for the E7 protein of a papillomavirus, a DNA fragment coding for the L1 protein of a papillomavirus, a DNA fragment coding for the L2 protein of a papillomavirus and a DNA fragment coding for interleukin-2, or
(6) a DNA fragment coding for the E6 protein of a papillomavirus, a DNA fragment coding for the E7 protein of a papillomavirus, a DNA fragment coding for the L1 protein of a papillomavirus, a DNA fragment coding for the L2 protein of a papillomavirus, a DNA fragment coding for the molecule B7.1 and a DNA fragment coding for interleukin-2.

15. Pharmaceutical composition according to Claim 7, **characterized in that** it comprises, as therapeutic agents, a first recombinant vector into which there is (are) inserted one or more DNA fragments coding for the said polypeptide(s) from the early region of a papillomavirus and one or more DNA fragments coding for the said polypeptide(s) from the late region of a papillomavirus and a second recombinant vector into which there is (are) inserted one or more DNA fragment(s) coding for the said polypeptide having immuno-stimulatory activity.

16. Pharmaceutical composition according to Claim 15, **characterized in that** the DNA fragments inserted into the first vector code for E6, E7, L1 and L2 and **in that** the DNA fragment inserted into the second vector codes for IL-2.

17. Pharmaceutical composition according to one of Claims 1 to 16, **characterized in that** it comprises a pharmaceutically acceptable carrier, allowing its administration by injection into humans or into animals.

18. Pharmaceutical composition according to one of Claims 1 to 17, as a medicament for the treatment or prevention of cancer of the neck of the uterus, of a dysplasia of the neck of low grade and of a papillomavirus infection.


### LISTE DE SEQUENCES

(1) INFORMATION GENERALE:
   (i) DEPOSANT:
      (A) NOM: TRANSGENE SA
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 27 91 11
   (ii) TITRE DE L' INVENTION: Composition pharmaceutique contre les tumeurs et infections a papillomavirus
   (iii) NOMBRE DE SEQUENCES: 16
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Human papillomavirus
      (B) SOUCHE: HPV-16
      (C) INDIVIDUEL ISOLE: oligonucleotide de synthese oTG5118 (E7 delete 21 a 26)
   (xi) DESCRIPTION DE LA SEQUENCE: SEQ ID NO: 1:
   TCTGAGCTGT CATTTAATTG AGTTGTCTCT GGTTGC 36
(2) INFORMATION POUR LA SEQ ID NO: 2:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Human papillomavirus
      (B) SOUCHE: HPV-16
      (C) INDIVIDUEL ISOLE: oligonucleotide de synthese oTG5377 (E6 delete 111 a 115)
   (xi) DESCRIPTION DE LA SEQUENCE: SEQ ID NO: 2:
   TGTCCAGATG TCTTTGCAGT GGCTTTTGAC AG 32
(2) INFORMATION POUR LA SEQ ID NO: 3:
   (1) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (il) TYPE DE MOLECULE: ADNC
   (iii) HYPOTHETIQUE: NON
   (iii) ANTI-SENS: NON
   (vi) ORIGINE:
      (A) ORGANISME: Homo sapiens
      (C) INDIVIDUEL ISOLE: oligonucleotide de synthese oTG6353 (PCR gene B7.1)
   (xi) DESCRIPTION DE LA SEQUENCE: SEQ ID NO: 3:
   TCAGCCCCTG AATTCTGCGG ACACTGTTAT ACAGG 35
(2) INFORMATION POUR LA SEQ ID NO: 4:
   (1) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: Homo sapiens
      (C) INDIVIDUEL ISOLE: oligonucleotide de synthese oTG6352 (PCR gène B7.1)
   (xi) DESCRIPTION DE LA SEQUENCE: SEQ ID NO: 4:
   TTGACCCTAA AGATCTGAAG CCATGGGCCA CAC 33
(2) INFORMATION POUR LA SEQ ID NO: 5:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Vaccinia virus
      (B) SOUCHE: Ankara modifiee
      (C) INDIVIDUEL ISOLE: oligonucleotide de synthese oTG7637 (PCR zone III)
   (xi) DESCRIPTION DE LA SEQUENCE: SEQ ID NO: 5:
   GGGGGGGAAT TCAGTAAACT TGACTAAATC TT 32
(2) INFORMATION POUR LA SEQ ID NO: 6:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Vaccinia virus
      (B) SOUCHE: Ankara modifiee
      (C) INDIVIDUEL ISOLE: oligonucleotide de synthese oTG7638 (PCR zone III)
   (xi) DESCRIPTION DE LA SEQUENCE: SEQ ID NO: 6:
   GGGGGGGGAT CCGAGCTCAC CAGCCACCGA AAGAGCAAT 39
(2) INFORMATION POUR LA SEQ ID NO: 7:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Vaccinia virus
      (B) SOUCHE: Ankara modifiee
      (C) INDIVIDUEL ISOLE: oligonucleotide de synthese oTG7635 (PCR zone III)
   (xi) DESCRIPTION DE LA SEQUENCE: SEQ ID NO: 7:
   GGGGGGGGAT CCGGAAAGTT TTATAGGTAG TT 32
(2) INFORMATION POUR LA SEQ ID NO: 8:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Vaccinia virus
      (B) SOUCHE: Ankara modifiee
      (C) INDIVIDUEL ISOLE: oligonucleotide de synthese oTG7636 (PCR zone III)
   (xi) DESCRIPTION DE LA SEQUENCE: SEQ ID NO: 8:
   GGGGGGGAAT TCTTTGTATT TACGTGAACG 30
(2) INFORMATION POUR LA SEQ ID NO: 9:
   (i) CARACTERISTIQUES DE LA SEQUENCE:
      (A) LONGUEUR: 78 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de synthese oTG10451
   (xi) DESCRIPTION DE LA SEQUENCE: SEQ ID NO: 9:
   CTTATACAGG GCGTACACTT TCCCTTCTCA ATCTCTCTCG AGTTGTATTT ATTTTCATTT     60
   TTTAAGTATA GAATAAAA                                                    78
(2) INFORMATION POUR LA SEQ ID NO: 10:
   (1) CARACTERISTIQUES DE LA SEQUENCE:
      (A) LONGUEUR: 86 paires de bases
      (B) TYPE: acide nucléique
      (C) NOMBRE DE BRINS: simple
      (D) CONFIGURATION: linéaire
   (il) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG10450
   (xi) DESCRIPTION DE LA SEQUENCE: SEQ ID NO: 10:
   AGCTTTTTAT TCTATACTTA AAAAATGAAA ATAAATACAA CTCGAGAGAG ATTGAGAAGG     60
   GAAAGTGTAC GCCCTGTATA AGGTAC                                           86
(2) INFORMATION POUR LA SEQ ID NO: 11:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Vaccinia virus
      (B) SOUCHE: Ankara modifiee
      (C) INDIVIDUEL ISOLE: oligonucleotide de synthese oTG7665 (PCR zone II)
   (xi) DESCRIPTION DE LA SEQUENCE: SEQ ID NO: 11:
   ATGGTACCGA ATTCCATCTA CCAATTCATC CAACAACAT 39
(2) INFORMATION POUR LA SEQ ID NO: 12:
   (i) CARACTERISTIQUES DE LA SEQUENCE:
      (A) LONGUEUR: 41 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Vaccinia virus
      (B) SOUCHE: Ankara modifiée
      (C) INDIVIDUEL ISOLE: oligonucleotide de synthese oTG7584 (PCR zone II)
   (xi) DESCRIPTION DE LA SEQUENCE: SEQ ID NO: 12:
   GGCTGCAGGA TCCGAGCTCA TCATGACGTC CTCTGCAATG G 41
(2) INFORMATION POUR LA SEQ ID NO: 13:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (il) TYPE DE MOLECULE: ADN (génomique)
   (iii) HYPOTHETIQUE: NON
   (iii) ANTI-SENS: NON
   (vi) ORIGINE:
      (A) ORGANISME: Vaccinia virus
      (B) SOUCHE: Ankara modifiee
      (C) INDIVIDUEL ISOLE: oligonucleotide de synthese oTG7639 (PCR zone II)
   (xi) DESCRIPTION DE LA SEQUENCE: SEQ ID NO: 13:
   GGGGGGGGAT CCTGTGAATC ATCCATTCCA CT 32
(2) INFORMATION POUR LA SEQ ID NO: 14:
   (i) CARACTERISTIQUES DE LA-SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Vaccinia virus
      (B) SOUCHE: Ankara modifiee
      (C) INDIVIDUEL ISOLE: oligonucleotide de synthese oTG7640 (PCR zone II)
   (xi) DESCRIPTION DE LA SEQUENCE: SEQ ID NO: 14:
   GGGGGGGAAT TCGTTACTAA ATTGCAAGGA AAT 33
(2) INFORMATION POUR LA SEQ ID NO: 15:
   (i) CARACTERISTIQUES DE LA SEQUENCE:
      (A) LONGUEUR: 69 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de synthese oTG10503
   (xi) DESCRIPTION DE LA SEQUENCE: SEQ ID NO: 15:
   CTCAAGTCAG TGTTGAGATG ATGCTTTGAC AACTCGAGTT TATTTTCATT TTTTAAGTAT 60
   AGAATAAAA 69
(2) INFORMATION POUR LA SEQ ID NO: 16:
   (i) CARACTERISTIQUES DE LA SEQUENCE:
      (A) LONGUEUR: 77 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG10502
   (xi) DESCRIPTION DE LA SEQUENCE: SEQ ID NO: 16:
   AGCTTTTTAT TCTATACTTA AAAAATGAAA ATAAACTCGA GTTGTCAAAG CATCATCTCA    60
   ACACTGACTT GAGGTAC                                                   77